# FASCICULE DE BREVET EUROPEEN

(11) **EP 2 222 294 B1**
(45) Date de publication et mention de la délivrance du brevet: **07.05.2014**
(21) Numéro de dépôt: 08850928.6
(22) Date de dépôt: 17.11.2008
(51) Int. Cl.: A61K 31/231, A61K 31/232, A23L 1/30, A23L 3/3472, A61K 8/37, A61P 17/18, A61K 31/23

(54) **COMPOSITIONS DE DÉRIVÉS POLYPHÉNOLIQUES STILBENIQUES ET LEURS APPLICATIONS POUR LUTTER CONTRE LES PATHOLOGIES ET LE VIEILLISSEMENT DES ORGANISMES VIVANTS**
ZUSAMMENSETZUNGEN MIT STILBENPOLYPHENOL-DERIVATEN UND IHRE VERWENDUNG ZUR BEKÄMPFUNG DER ALTERUNG VON LEBENDEN ORGANISMEN UND KRANKHEITEN MIT EINFLUSS DARAUF
COMPOSITIONS COMPRISING STILBENE POLYPHENOL DERIVATIVES AND USE THEREOF FOR COMBATING THE AGEING OF LIVING ORGANISMS AND DISEASES AFFECTING SAME

(30) Priorité: 15.11.2007 FR 0708020
(43) Date de publication de la demande: 01.09.2010
(73) Titulaire: Caudalie, 75008 Paris (FR)
(72) Inventeur: VERCAUTEREN, Joseph, F-34170 Castelnau-le-lez (FR)
(74) Mandataire: Peaucelle, Chantal
(86) Numéro de dépôt international: PCT/IB2008/054818
(87) Numéro de publication internationale: WO 2009/063440

(56) Documents cités:
- WO-A-01/91695
- WO-A-91/09595
- FR-A- 2 766 176
- FR-A- 2 772 613
- M.G. SARPIETRO ET AL.: "INTERACTION OF RESVERATROL AND ITS TRIMETHYL AND TRIACETY DERIVATIVES WITH BIOMEMBRANE MODELS STUDIED BY DIFFERENTIAL SCANNING CALORIMETRY" JOURNAL OF AGRICULTURAL FOOD CHEMISTRY, vol. 55, 2 mai 2007 (2007-05-02), pages 3720-3728, XP002478612

## Description

L'invention a pour objet des compositions de dérivés polyphénoliques stilbéniques pour prévenir et lutter contre la plupart des pathologies et le vieillissement des tissus et des organismes vivants. Elle concerne également un procédé de préparation de ces compositions ainsi que leurs applications, notamment, dans les domaines cosmétique, diététique et thérapeutique.

Depuis plus d'un demi-siècle, s'est développée l'hypothèse selon laquelle le vieillissement de l'organisme humain résulte de l'accumulation de multiples dégâts causés aux tissus par des espèces radicalaires ou de réactivités chimiques oxydantes.

Au milieu des années 50, après de nombreux travaux sur le caoutchouc, le chimiste Harman constatait qu'empêcher la formation de radicaux libres était le moyen le plus sûr de lutter contre sa dégradation et son craquèlement. Par analogie, il suggère alors que le vieillissement des tissus chez l'homme (apparition de rides sur la peau, par exemple) serait dû à la formation « anormale » au sein des cellules, d'espèces chimiques très réactives, et notamment, de radicaux libres et aux suites de réactions qu'elles déclenchent.

Des espèces oxygénées réactives (EOR) sont formées au niveau mitochondrial par « transfert » incontrôlé d'électron(s) à l'oxygène (EOR : anion superoxyde, peroxydes, peroxynitrites, radicaux libres, ...).

Ces EOR se propagent ensuite aux autres compartiments cellulaires ou au cytoplasme, en fonction de leur hydro/liposolubilité, où elles créent des dégâts considérables.

Dans un tel contexte, la recherche de substances actives pour lutter contre le vieillissement s'est faite, durant ces dernières décennies, sur la base de leur capacité à briser les réactions d'oxydation en chaîne, c'est-à-dire à prévenir le stress oxydant. Effectivement, toute substance capable d'interagir avec les EOR, en diminue les effets délétères et, sur le plus long terme, aura un impact positif sur la santé et, pour les mêmes raisons, ralentira le vieillissement comme le développement des principales pathologies. Il s'agit de piégeurs de radicaux libres (aptitude à délivrer un seul électron à la fois) et/ou antioxydants (transfert de deux électrons en même temps) tels que les vitamines (E et C) et les polyphénols.

Toutefois, les dégâts qui provoquent le vieillissement de l'organisme ou qui accompagnent les principales pathologies, ne seraient pas seulement la conséquence d'un mauvais contrôle du flux d'électrons dus aux « fuites » du métabolisme mitochondrial et des EOR intracellulaires, mais impliqueraient également d'autres sources d'effets délétères potentiels faisant intervenir la « réaction de Maillard » et le stress carbonylé.

Dans le stress carbonylé, la fonction carbonyle (aldéhyde) du glucose exerce ses propriétés électrophiles vis à vis des résidus nucléophiles des protéines (amines, thiols, ...) : c'est le point de départ du stress carbonylé qui s'amplifie par formation de propagateurs.

Les espèces chimiques créées, ou produits de glycation, sont considérées comme des produits de fins : ce sont des AGEs pour « Advanced Glycated End-Products », dans lesquels, le glucose ou ses fragments sont liés aux résidus aminoacides de manière irréversible.

Les réactions de Maillard qui se produisent augmentent, dans le même temps, la capacité réductrice des sucres et de leurs dérivés. Les composés dicarbonylés qui se forment, acquièrent une oxydabilité bien plus forte encore que leurs précurseurs et transfèrent facilement leurs électrons à l'oxygène, par exemple. A partir de l'anion superoxyde formé initialement, une même suite d'EOR que dans le cas du stress intracellulaire est produite. Ainsi, le stress carbonylé se double-t-il d'un second type de stress oxydant.

A la différence des mécanismes évoqués précédemment pour les EOR d'origine mitochondriale, ce nouveau stress oxydant se produit à l'extérieur des cellules, au sein de la matrice extracellulaire. Il concerne donc les aminoacides ou les résidus des protéines de cette matrice, et notamment, les fibres de collagène et d'élastine. Ce stress oxydant, particulièrement important du fait que les systèmes de protection enzymatiques ne sont pas aussi efficaces que ceux situés dans la cellule, débouche sur une augmentation des phénomènes d'alkylation qui s'additionnent aux produits de glycation et de glycoxydation, issus du stress carbonylé.

Ainsi, le stress carbonylé, doublé d'un stress oxydant extracellulaire est-il au moins aussi important que le stress oxydant intracellulaire dans le développement du vieillissement et la mise en place des altérations tissulaires accompagnant les principales pathologies.

L'étude par les inventeurs des phénomènes conduisant au vieillissement des tissus les ont ainsi amenés à une prise en compte plus étendue des mécanismes biochimiques qui en sont responsables et à dégager de nouveaux concepts permettant de définir de nouvelles cibles biologiques d'actions complémentaires pour les combattre plus efficacement.

Leurs recherches ont alors conduit à modifier la structure de polyphénols à propriétés anti-oxydantes et piégeuse de radicaux libres, tels que ceux constitutifs d'extraits végétaux, pour leur conférer de plus grandes aptitudes à piéger également les stresseurs carbonylés.

L'invention a donc pour but de fournir de nouvelles compositions de dérivés de polyphénols constituant des polyphénols suractivés qui sont à la fois capables d'agir avec une grande efficacité sur un plus grand nombre de cibles biologiques (stress oxydants et carbonylé) et sont stabilisés.

L'invention a également pour but de fournir un procédé permettant d'obtenir de tels dérivés polyphénoliques à partir de polyphénols d'extraits végétaux.

Selon encore un autre aspect, l'invention vise la mise à profit des propriétés de ces compositions polyphénoliques de type phloroglucinoliques, en cosmétologie, diététique et en thérapeutique.

Les compositions de dérivés de polyphénols de l'invention sont caractérisées en ce que lesdits polyphénols renferment des monomères, des oligomères ou des polymères d'unités répondant à la formule (I) :

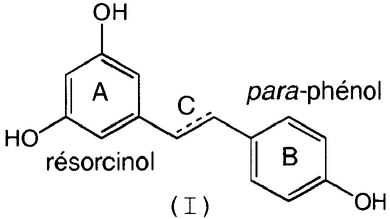

Ces unités se caractérisent par la présence simultanée d'un noyau de type résorcinol (noyau A) et d'un noyau de type para-phénol (noyau B), reliés entre eux par un lien carboné tel que C. Dans le cas le plus simple, les deux noyaux A et B sont confondus et le segment C est inexistant, ce qui correspond au cas du phloroglucinol de formule (II)

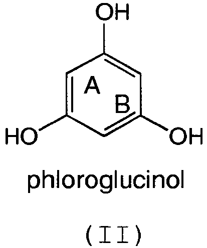

Les noyaux A et B de ces unités sont le plus souvent distincts, et le segment C est constitué de 2 carbones qui sont soit hybridés sp2 et forment un vinyle : c'est le cas du resvératrol de formule (III)

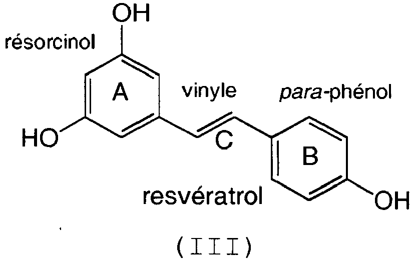

Le segment C peut aussi bien être constitué de carbones hybridés sp3 et servir, notamment, de point d'attachement entre les monomères pour former les polymères.

Lesdits dérivés sont suractivés, en ce qui concerne leur pouvoir nucléophile, par alkylation d'au moins une fonction phénolique de chaque unité et stabilisés par estérification par des mélanges d'acides gras majoritairement insaturés (AGI) de toutes les autres restées libres.

De manière générale, les substitutions spécifiques des dérivés des compositions de l'invention conduisent à une modulation de leur activité et les rendent capables d'inhiber en même temps et spécifiquement les mécanismes principaux impliqués dans les pathologies majeures et le vieillissement évoqués ci-dessus.

Avantageusement, le nombre de groupes -*O*-alkyles par molécule n'égale pas le nombre d'hydroxyles présents en moyenne par molécule et, de préférence, il est de 1 ou 2.

Le ou les groupes alkyles sont plus particulièrement des groupes à effet électrodonneur : méthyles, isopropyles ou *tert*-butyles, pour renforcer au maximum la nucléophilie des noyaux aromatiques et, en conséquence, leur aptitude à piéger les stresseurs carbonylés.

La stabilisation efficace est obtenue par formation d'esters d'AGI entre les fonctions phénoliques restées libres et les acides gras issus d'huiles végétales à acides gras majoritairement insaturés (AGI). Les huiles sont choisies pour leur impact favorable sur la santé. Avantageusement, les actifs obtenus renferment alors des proportions d'acides gras insaturés identiques à celles des huiles dont ils proviennent.

Lesdits esters comprennent de préférence les mélanges de radicaux acyles R des acides gras d'huile d'olive (*Olea europea*) ou d'huile de pépins de raisin (*Vitis vinifera*).

Il s'agit plus spécialement de radicaux R d'acides gras saturés (AGS = ac. stéarique ; 7-8%), d'acides gras monoinsaturés (AGMI = acide oléique ; 55-75%) et d'acides gras polyinsaturés essentiels (AGPI ; 15-18%) : diinsaturés (ac. linoléiques) et triinsaturés (ac. linoléniques) des séries ω-6 et ω-3, présents dans les dérivés de l'invention dans des proportions identiques à celles des huiles qui exercent un bénéfice maximal sur la santé, selon les données issues de l'épidémiologie.

Cette stabilisation permet par ailleurs de protéger les polyphénols stilbéniques suractivés d'une destruction prématurée certaine (oxydation à l'air ou à la lumière), tout en leur donnant un caractère lipophile afin d'augmenter leurs chances d'être résorbés. Voir à ce propos FR 2 766 176 et FR 2 772 613.

Avantageusement, cette stabilisation est cependant temporaire, et n'est plus efficace quand les dérivés sont placés en situation d'agir, afin de leur restituer tout leur pouvoir antioxydant. Elle doit donc être réversible par simple action des systèmes biologiques auxquels les groupes stabilisants sont alors exposés, et notamment, les enzymes telles que lipases, estérases ou protéases.

Plus spécifiquement, l'invention vise des compositions caractérisées en ce que lesdits dérivés répondent à la formule (IV) 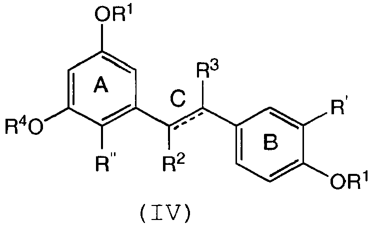 dans laquelle
- R¹ est un radical alkyle, ou un radical acyle d'un acide gras d'une huile végétale, représenté par R tel que défini ci-dessus,
- R² est un hydrogène ou le point de jonction en R" ou à R² d'une autre unité,
- -R³ est un hydrogène ou le point de jonction en R" ou en R⁴ d'une autre unité,
- R⁴ est un radical alkyle, ou un radical acyle d'un acide gras d'une huile végétale, représenté par R tel que défini ci-dessus ou le point de jonction en R³ d'une autre unité.
- R" représente H ou le point de jonction en R² ou en R³ d'une autre unité,
- R' est un hydrogène ou un radical O-acyle d'un acide gras d'une huile végétale, représenté par R tel que défini ci-dessus
et les diastéréoisomères et les régioisomères de ces motifs.

À titre d'exemple, on peut donner les dimère (epsilon-viniférine) et trimère (miyabénol C), de formules (V) et (VI) :

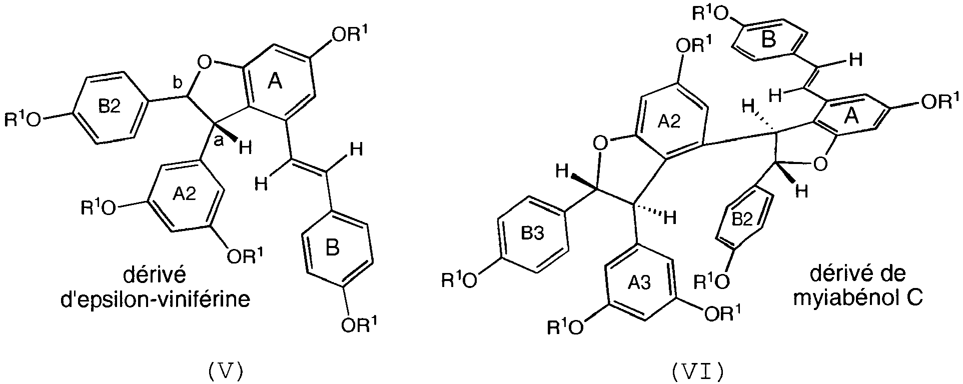

Selon une disposition préférée de l'invention, les dérivés définis ci-dessus correspondent à des dérivés alkylés, puis stabilisés d'extraits végétaux. Ils présentent donc les structures des polyphénols présents en mélange dans ces extraits végétaux.

Ainsi, lesdits extraits végétaux sont essentiellement constitués de dérivés du resvératrol, ce dernier répondant à la formule (III)

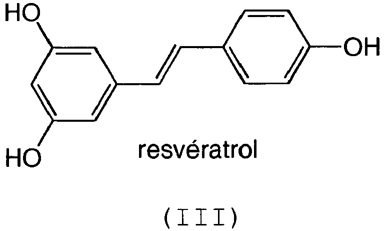

Dans un premier groupe de cette famille, il s'agit plus particulièrement d'extraits de vigne.

L'invention vise tout spécialement les dérivés d'extraits de sarments et/ou de rafles de vigne (*Vitis vinifera*).

L'invention vise ainsi des compositions de dérivés de polyphénols d'extraits de sarments de vigne, ces extraits comprenant des quantités importantes de dérivés polyphénoliques constituant, comme indiqué plus haut, des équivalents vinylogues du phloroglucinol. Il s'agit notamment de polyphénols de formules III, VII, VIII, IX et X ci-dessous correspondant respectivement au resvératrol, picéatannol, epsilon-viniférine, pallidol, miyabénol C.

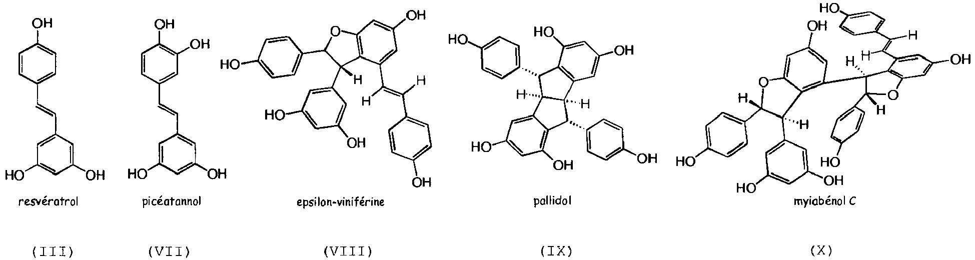

Dans un deuxième groupe de ladite première famille, il s'agit de dérivés d'extraits de Polygonum (*Polygonum cuspidatum.*

Dans un troisième groupe, il s'agit de dérivés d'extraits de fruits, par exemple de mûriers (*Morus sp*).

Les compositions de dérivés de polyphénols de l'invention sont avantageusement obtenues selon un procédé comprenant la réaction des compositions de polyphénols des extraits végétaux définis ci-dessus
- dans une première étape, avec un agent d'alkylation dans des conditions permettant de substituer l'hydrogène d'au moins 1 groupe OH phénolique par molécule, de préférence de 1 à 2, par un groupe alkyle, et
- dans une deuxième étape, avec un agent d'acylation, notamment, un anhydride ou un chlorure d'acide, dans des conditions permettant de substituer l'hydrogène des groupes -OH, encore libres après alkylation, par un mélange de radicaux acyles -COR libérés par l'agent d'acylation, R étant tel que défini ci-dessus.

La réaction d'alkylation fait appel à des réactifs disponibles commercialement, tels que des halogénures (idodures, bromures, ...), ou des esters sulfuriques à raison d'un et demi équivalent chimique). Ils sont additionnés lentement à une solution de l'extrait polyphénolique dans un solvant aprotique (acétone anhydre, par exemple), et en présence d'une base minérale (carbonate de potassium, ...), porté au reflux, sous agitation et atmosphère inerte (azote, argon, idéalement).

La réaction d'alkylation est arrêtée, après refroidissement, par ajout d'un acide dilué (chlorhydrique, par exemple) jusqu'à obtention d'un pH acide. L'agitation est poursuivie pendant 45 min supplémentaires, environ. Le milieu réactionnel est concentré sous vide (évaporation du solvant). La phase aqueuse est extraite par un égal volume de solvant non miscible (acétate d'éthyle, dichlorométhane, ...), qui est lui-même lavé par deux volumes équivalents d'eau distillée (jusqu'à neutralité). Cette phase organique est séchée sur sulfate de sodium anhydre, puis filtrée et évaporée sous pression réduite pour abandonner le résidu des polyphénols alkylés.

L'agent d'acylation est préparé à partir d'une huile végétale selon un procédé comprenant :
- la saponification des glycérides d'une huile végétale, suivie d'une acidification,
- une activation par déshydratation dans le cas où l'agent d'acylation est un anhydride d'acide, ou par chloruration, dans le cas où il s'agit d'un chlorure d'acide, mais d'autres dérivés conférant le même effet d'activation peuvent être utilisés (transestérification, acylation enzymatique, selon les cas).

La réaction de saponification est effectuée en phase aqueuse en présence d'un agent alcalin comme l'hydroxyde de potassium en quantité au moins stoechiométrique, de préférence à la température de reflux. La solution est alors amenée à pH acide par addition d'acide minéral, puis extraite par un solvant organique afin d'isoler le mélange des acides libres formés lors de la réaction.

La réaction de déshydratation se déroule à reflux, en présence de solvant capable de créer un azéotrope avec l'eau, afin de permettre son élimination, au fur et à mesure de sa formation. On utilise par exemple le toluène et on piège l'eau par un système type « Dean Stark ».

La réaction de chloruration est menée en présence de solvant capable de dissoudre les acides gras libres. Elle est catalysée par une base de Lewis et réalisée par addition lente de l'agent de chloruration, à température contrôlée, proche de 0°C. Quand l'addition est terminée, l'agitation est prolongée à la température ambiante, puis le milieu réactionnel est concentré par évaporation sous vide, et les chlorures sont purifiés par distillation.
Avantageusement:
- On utilise comme solvant de la chloruration, du dichlorométhane ou du chloroforme, par exemple, à condition qu'il ne soit pas stabilisé par un alcool,
- L'agent de chloruration est, par exemple, le chlorure de thionyle ou le chlorure d'oxalyle,
- Le catalyseur peut être le diméthylformamide,
- La purification des chlorures d'acyles a lieu par distillation sous vide poussé, dans un « four à boules » (Kugelrohr).

La réaction d'acylation est le plus souvent effectuée en présence d'un solvant permettant une solubilisation, même partielle, des composés polyphénoliques alkylés résultant de la réaction d'alkylation décrite ci-dessus.

Des solvants appropriés sont choisis parmi des dérivés halogénés comme le dichlorométhane, le chloroforme ou le 1,2-dichloroéthane, ou des dérivés azotés comme la pyridine, ou même, l'hexane, selon les composés alkylés à dissoudre.

Les dérivés polyphénoliques alkylés, en solution dans le solvant de réaction choisi et avantageusement additionnés d'un agent de catalyse basique (par exemple, la triéthylamine ou la pyridine), sont placés sous agitation et atmosphère inerte (argon, azote).

Deux équivalents d'anhydrides ou de chlorures d'AG, tels que préparés ci-dessus, sont utilisés comme agents d'acylation. Ils sont additionnés goute à goutte, en solution dans le solvant de la réaction, s'il ne s'agit pas de pyridine seule. Dans le cas où la pyridine est à la fois le solvant et le catalyseur basique, on procède à une addition « inverse ». C'est la solution des dérivés polyphénoliques qui est additionnée goutte à goutte aux acylpyridiniums préformés.

Une alternative qui peut s'appliquer, consiste à ajouter sous agitation énergique, une phase aqueuse basique (Na₃PO₄, K₃PO₄) à la solution organique (CHCl₃, CH₂Cl₂) des dérivés polyphénoliques alkylés et des agents d'acylation, réalisant ainsi les conditions de Schotten-Baumann.

Quelle que soit la procédure adoptée, la réaction est réalisée de préférence à température ambiante, sur une durée d'environ 7 à 8 heures.

Les dérivés estérifiés ainsi formés sont purifiés par addition d'eau acidulée (HCl, qs pH acide), puis par plusieurs lavages de la phase organique à l'eau distillée. Après séchage sur sulfate de sodium, la solution est filtrée, puis évaporée à siccité pour livrer les actifs flavonoïdiques alkylés et stabilisés.

Les actifs à double potentialité de l'invention, capables de piéger à la fois les EOR, quelle que soit leur origine intra ou extracellulaire et de piéger les composés dicarbonylés (anti-glycation et anti-AGEs), présentent un grand intérêt comme moyens de lutte les plus complets et les plus efficaces à ce jour contre le vieillissement cutané.

Les compositions de l'invention sont donc particulièrement appropriées pour l'élaboration de préparations cosmétiques.

Dans ces préparations, les compositions sont associées à des véhicules appropriés pour un usage externe. De manière avantageuse, leur caractère liposoluble favorise leur incorporation dans les formes galéniques habituellement utilisées en cosmétique.

L'invention vise donc des compositions cosmétiques caractérisées en ce qu'elles renferment une quantité efficace pour lutter contre le vieillissement de la peau, d'une ou plusieurs compositions de dérivés de polyphénols stilbéniques telles que définies ci-dessus en association avec des véhicules inertes appropriés pour un usage externe.

Ces compositions se présentent sous une forme appropriée pour une administration par voie topique telle que crème, pommade, émulsion, gel, liposomes, lotion.

Elles renferment de 0,5 à 5 % de produit actif, de préférence de 2 à 3 %.

L'invention concerne également une méthode pour prévenir le vieillissement de la peau, caractérisée par l'application sur la peau ou l'ingestion d'une ou plusieurs compositions cosmétiques telles que définies ci-dessus.

Selon un autre aspect de grand intérêt, les compositions de l'invention sont utilisables en diététique. Grâce notamment à leurs propriétés anti-radicalaires et piégeuses de composés carbonylés, elles assurent une meilleure conservation des aliments. De plus, elles constituent généralement un apport de facteur vitaminique. Elles sont donc ajoutées avec avantage aux boissons, par exemple aux jus de fruits, boissons toniques, aux produits laitiers et dérivés comme le beurre.

Elles sont également utilisables telles quelles sous forme liquide, ou encore en granulés ou analogues, gels ou sous forme de pâte, par exemple incorporées dans des confiseries comme les pâtes de fruits, bonbons, pâtes à mâcher.

Les propriétés des compositions de l'invention sont également avantageusement mises à profit pour une utilisation comme médicaments.

L'invention vise ainsi des compositions pharmaceutiques, caractérisées en ce qu'elles renferment une quantité thérapeutiquement efficace d'au moins une composition telle que définie ci-dessus, en association avec un véhicule pharmaceutiquement acceptable.

Ces compositions se présentent avantageusement sous une forme appropriée pour une administration notamment par voie orale, topique ou parentérale.

Ainsi, pour une administration par voie orale, les compositions se présentent plus particulièrement sous forme de comprimés, gélules, solutions ou sirops.

Pour une administration par voie topique, les compositions se présentent sous forme de crème, pommades, gels, patchs ou lotions.

Pour une administration par voie parentérale, les compositions de présentent sous forme de solution injectable stérile ou stérilisable.

D'autres caractéristiques et avantages de l'invention sont donnés, à titre illustratif, dans les exemples qui suivent dans lesquels il est fait référence aux figures 1 à 11, qui représentent, respectivement:
- figure 1 : le spectre IR-FT en mode ATR, des resvératrols monoalkylés (méthylés) par l'iodure de méthyle,
- Figure 2 : le spectre RMN 2D HMBC ¹H-¹³C (500 MHz) des resvératrols monoalkylés (méthylés) par l'iodure de méthyle,
- Figure 3 : le spectre IR-FT des resvératrols monoalkylés (méthylés) par le DMS,
- Figure 4 : le spectre RMN 2D HMBC ¹H-¹³C (500 MHz) des resvératrols monoalkylés (méthylés) par l'iodure de méthyle,
- Figure 5 : le spectre IR-FT des acides gras issus de la saponification d'une huile d'olive « vierge », en mode ATR,
- Figure 6 : le chromatogramme en phase gazeuse, détecté par spectrométrie de masse (GC-DSQ2) des esters méthyliques préparés à partir des chlorures d'AG d'olive,
- Figure 7 : le spectre IR-FT des chlorures d'AG d'olive (en mode ATR),
- Figure 8 : le spectre RMN du proton à 500 MHz (CDCl₃) des chlorures d'AG d'olive,
- Figure 9 : le spectre IR-FT des polyphénols stilbénoïdiques de sarments de vigne alkylés et stabilisés par des AG d'huile d'olive,
- Figure 10 : le spectre de RMN 2D HMBC ¹H-¹³C (500 MHz, CDCl₃) des polyphénols stilbénoïdiques de sarments de vigne alkylés et stabilisés par des AG d'huile d'olive.

### Exemple 1 : O-alkylation du phloroglucinol

1,560 g de phloroglucinol (12,3 mmoles) sont dissous dans 20 mL d'acétone anhydre, dans un bicol surmonté d'un réfrigérant. Sous agitation et atmosphère d'argon, en présence de 1,685 g (12,3 mmoles, 2 éq. chimiques) de carbonate de potassium (K₂CO₃), on ajoute 766 microlitres d'iodure de méthyle (= 1,746 g ; d = 2.28 g/mL à 25 °C), soit 12,3 mmoles = 1 équivalent molaire par rapport au resvératrol. La réaction est portée au reflux pendant 3 heures.

Le milieu réactionnel est filtré sur fritté n°4, pour éliminer le K₂CO₃ et l'acétone est évaporée sous vide. Le résidu est repris dans 15 mL d'acétate d'éthyle. La phase organique, lavée par 2 fois 15 mL d'eau distillée, séchée sur sulfate de sodium, filtrée et évaporée à siccité, abandonne un résidu de 1357 mg, identifié au 5-méthoxyrésorcinol (rendement brut = 89 % ; pm = 124) sur la base de ses constantes spectrales : RMN ¹H, acetone-*d*6, 500 MHz, δ ppm : 5,95 (1H, d); 5,90 (2H, d); 3,65 (3H, s, Ch₃). RMN ¹³C, acetone-*d*6*,* 125 MHz, δ ppm : 167,2 (s); 164,22 (2 s); 100,61 (d); 98,26 (2 d); 59,6 (quad.).

### Exemple 2 : O-alkylation du resvératrol

450 mg de resvératrol (1,97 mmoles) sont dissous dans 10 mL d'acétone anhydre, dans un bicol surmonté d'un réfrigérant. Sous agitation et atmosphère d'argon, en présence de 270 mg (1,97 mmole, 2 éq. chimiques) de carbonate de potassium (K₂CO₃), on ajoute 123 microlitres d'iodure de méthyle (= 280 mg ; d = 2.28 g/mL à 25 °C), soit 1,97 mmoles = 1 équivalent molaire par rapport au resvératrol. La réaction est portée au reflux pendant 3 heures.

Le milieu réactionnel est filtré sur fritté n°4, pour éliminer le K₂CO₃ et l'acétone est évaporée sous vide. Le résidu est repris dans 15 mL d'acétate d'éthyle. La phase organique, lavée par 2 fois 15 mL d'eau distillée, séchée sur sulfate de sodium, filtrée et évaporée à siccité, abandonne un résidu de 548 mg (rendement brut = 91,6 %, sur la base de resvératrols monométhylés, pm = 304).

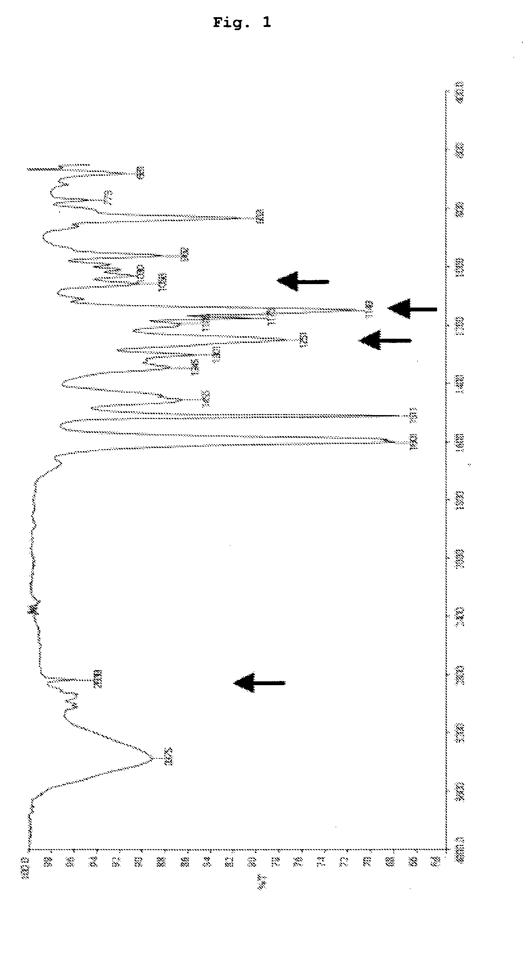

L'étude en spectroscopie IR-FT de cet extrait de resvératrols-O-méthylés permet d'établir les caractéristiques communes à tous ces dérivés méthylés, y compris les plus complexes des extraits polyphénoliques stilbénoïdiques de végétaux, repérées par des flèches sur le spectre (Fig. 1) : bandes à 2838 (CH) et 1251, 1143 et 1058 cm⁻¹ (éthers).

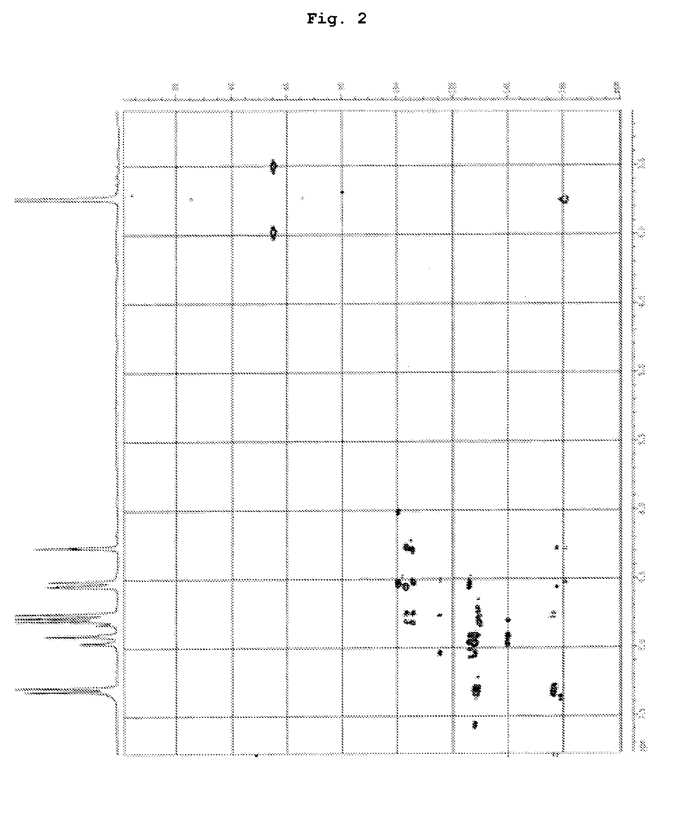

Ce mélange de resvératrols monométhylés est caractérisé en RMN par les corrélations, diagnostiques de l'alkylation, entre des carbones oxygénés aromatiques du resvératrol (δ = 160 ppm) et les protons des éthers méthyliques (δ = 3,8 ppm). Le spectre de RMN 2D HMBC (Fig. 2), montre des corrélations entre des carbones aromatiques oxygénés (de 155 à 162 ppm) et les protons des éthers méthyliques, résonnant à une fréquence centrée sur 3,8 ppm.

### Exemple 3 : Étape d'O-alkylation de polyphénols stilbéniques

On opère sur 10,08 g (44 mmoles) d'extrait de polyphénols stilbénoïdiques tel qu'obtenu selon le procédé du brevet FR 2 766176, sont dissous dans 50 mL d'acétone. 12,25 g de K₂CO₃ activé (88 moles = 4 éq. chimiques), puis 3,15 mL (33 mmoles, 1,5 éq. chimiques) de l'agent alkylant, ici le diméthylsulfate (DMS), sont ajoutés sous agitation et atmosphère d'argon.

Le calcul des équivalents chimiques se fait en comptant une « moyenne » de 3 résidus hydroxyles par unité de resvératrol. Ainsi, on considère que chaque tranche de 228 g d'extrait correspond à "1 mole de resvératrol", et possède trois fonctions phénoliques dont une seule doit être transformée en éther d'alkyle. L'équivalent chimique du réactif d'alkylation est donc égal au tiers du nombre de moles d'extrait de resvératrol mis en oeuvre, en considérant que le poids moléculaire est de 228.

La solution limpide obtenue est portée au reflux pendant 7 heures et la réaction est refroidie. Après addition d'une solution d'acide chlorhydrique dilué, jusqu'à obtention d'un pH acide (220 mL), l'agitation est poursuivie pendant 45 min supplémentaires. Le milieu réactionnel est concentré sous vide (évaporation de l'acétone). La phase aqueuse résiduelle est extraite par un égal volume d'acétate d'éthyle, qui est lavé par deux fois 200 mL d'eau distillée (jusqu'à neutralité de l'eau de lavage). Cette phase organique est ensuite séchée sur sulfate de sodium anhydre, filtrée et évaporée sous pression réduite pour abandonner le résidu des polyphénols stilbénoïdiques alkylés (9,923 g ; rendement brut = 93,2%, pm moyen = 242).

Dans le cas préféré, où chaque molécule de l'extrait initial, subit une seule méthylation par unité stilbénoïdique (« resvératrol »), on obtient un mélange des différents régio-et stéréo-isomères possibles, tels que les monomères et dimères figurés ci-dessous.

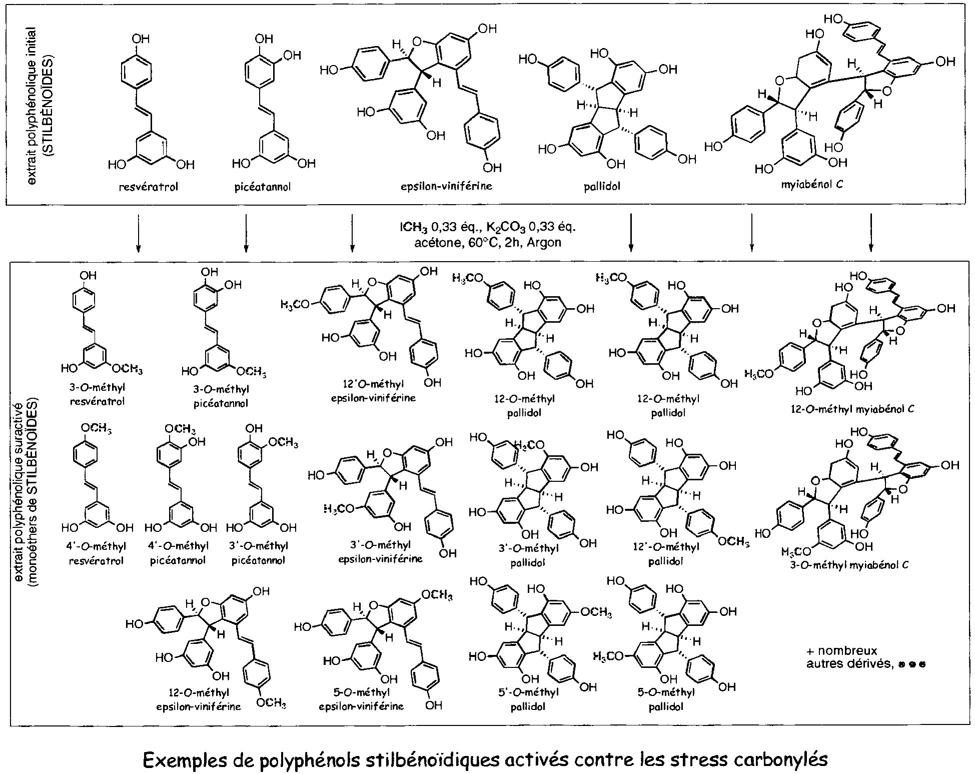

Généralement, les diverses fonctions phénoliques de chacune des molécules réagissent cependant avec des cinétiques différentes. Pour le resvératrol, par exemple, les proportions entre les différents isomères de position sont les suivantes :

| | |
|---|---|
| resvératrol | 6 % |
| 3-*O*-méthyl-resvératrol | 8 % |
| 4'-*O*-méthyl-resvératrol | 13 % |
| 3,4'-di-*O*-méthyl-resvératrol et 3,5-di-*O*-méthyl-resvératrol | 23 % |
| 3,4',5-tri-*O*-méthyl-resvératrol | 13 % |

Il en résulte une grande diversification des actifs stilbénoïdiques suractivés qui sont constitués des dérivés monométhylés de la figure ci-dessus, mais ils sont accompagnés de manière minoritaire cependant, des isomères di- et tri-méthylés, possibles.

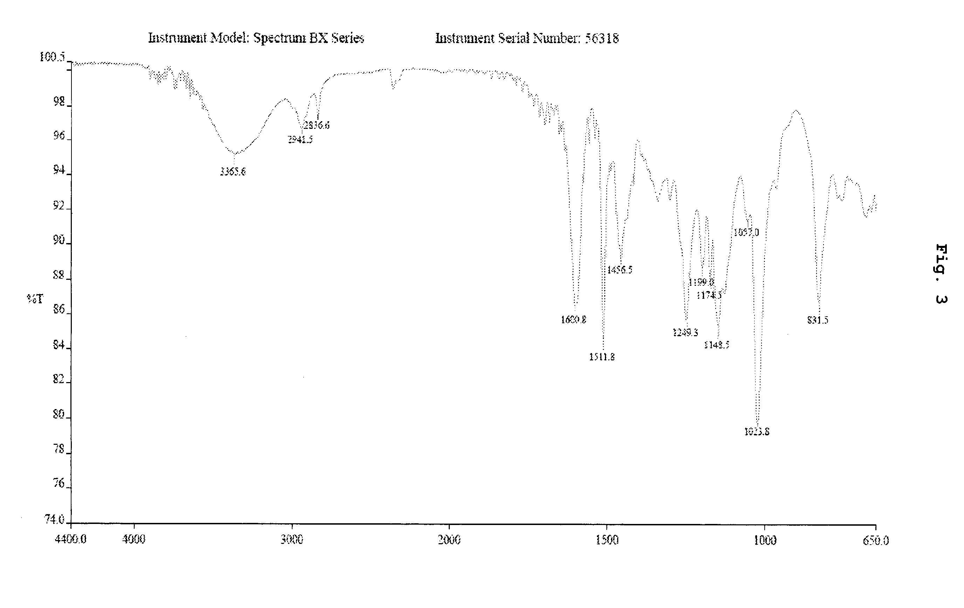

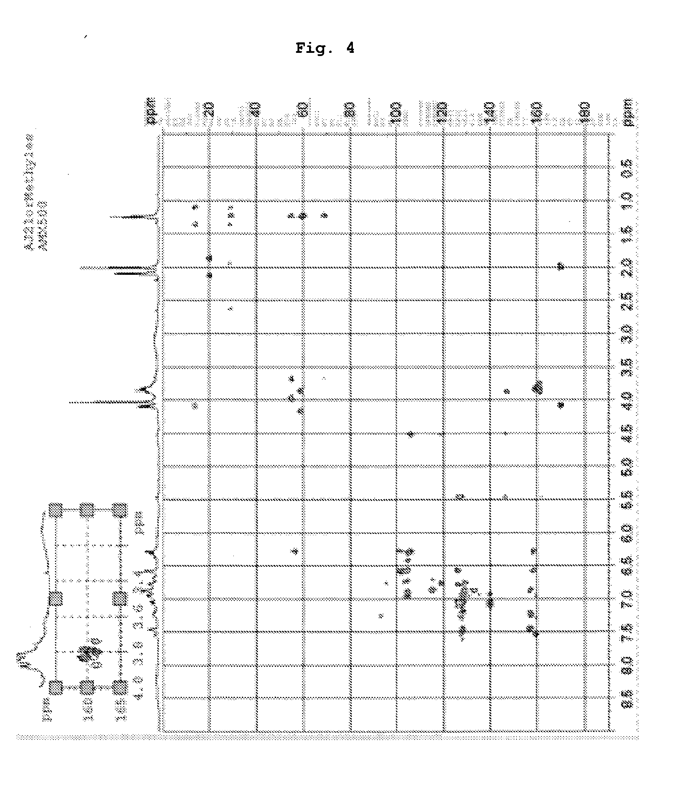

Comme pour l'exemple précédent, les structures alkylées (méthylées), de ces composés stilbénoïdiques, sont déduites de l'analyse de leurs différents spectres :
- La présence d'éthers méthyliques phénoliques se traduit en IR (Fig. 3), notamment, par l'apparition de bandes d'absorption entre 2974 et 2836 cm⁻¹ caractéristiques des C-H de méthyles (élongation) et, entre 1040 et 1235 cm⁻¹, celles caractéristiques des fonctions (C-O) éthers.
- Le spectre RMN 2D HMBC (Fig. 4), montre des corrélations entre des carbones aromatiques oxygénés (de 155 à 160 ppm) et les protons des éthers méthyliques, résonnant à une fréquence centrée sur 3,8 ppm.

### Exemple 4 : Préparation des agents acylants

### Étape n° 1 : saponification de l'huile d'olive :

50,46 g d'huile d'olive « vierge » (57 mmoles, = "171 eq") placés dans un ballon équipé d'un condenseur, sont additionnés de 16,08 g d'hydroxyde de potassium (285 mmoles, 1,67 eq), en solution dans 2,5 mL d'éthanol et 50 mL d'eau. La réaction est portée au reflux pendant 5 heures. Elle est agitée encore pendant 14 h, à température ambiante.

Après avoir étendu la solution résultante par 300 mL d'eau, on ajoute de l'acide chlorhydrique au dixième (3,7% ; p/v), jusqu'à obtenir un pH acide de la phase aqueuse (250 mL environ). Le contenu du ballon, qui comporte un « insoluble » pâteux en surface, est alors transféré en ampoule à décanter et extrait par 700 mL d'hexane. La phase organique est séparée puis lavée par 2 fois 300 mL d'eau distillée (obtention d'un pH neutre de cette phase aqueuse).

La phase organique est séchée sur sulfate de sodium, filtrée sur fritté n°4, puis évaporée pour livrer un résidu de 42,9 g (rendement brut = 88,8%).

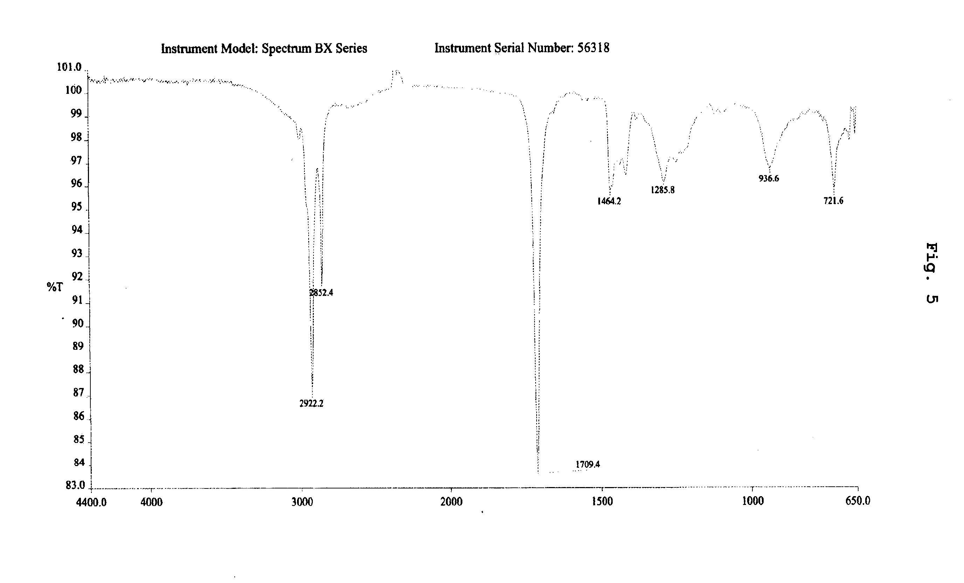

Le spectre infrarouge enregistré en mode ATR avec transformée de Fourier (Fig. 5) montre une bande caractéristique des acides organiques libres à 1709 cm⁻¹, en même temps que la disparition des bandes esters de l'huile de départ.

### Étape n° 2 : Activation des acides gras issus de la saponification de l'huile d'olive par formation de chlorures :

La solution de 41,5 g d'acides gras libres (147,1 mmoles) issus de l'étape n° 1, dans 232 ml de chloroforme (stabilisé sur amylène), est agitée, sous atmosphère d'argon, dans un ballon refroidi par un bain de glace. Par le biais d'une ampoule à brome, on introduit goutte à goutte 13,8 mL de chlorure d'oxalyle (162 mM = 1,1 eq), sur une période de 30 minutes. On introduit 1 mL de diméthylformamide (DMF), et l'agitation est poursuivie sur bain de glace pendant 5 minutes. La concentration sous pression réduite du mélange réactionnel (chloroforme et chlorure d'oxalyle en excès), fournit alors 44,3 g de résidu huileux, légèrement coloré en jaune (Rdt brut = 100%).

Par distillation dans un four à boules (kugelrhor), sous vide important (2 mm Hg), ce résidu est débarrassé de sa coloration (liquide incolore), en collectant les fractions qui distillent de 178 à 195°C.

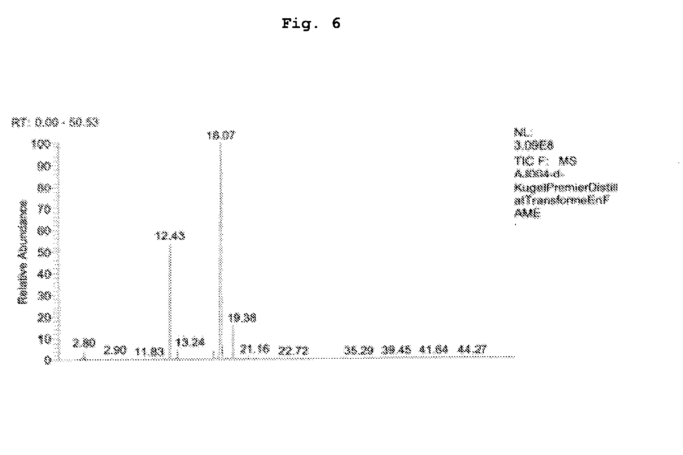

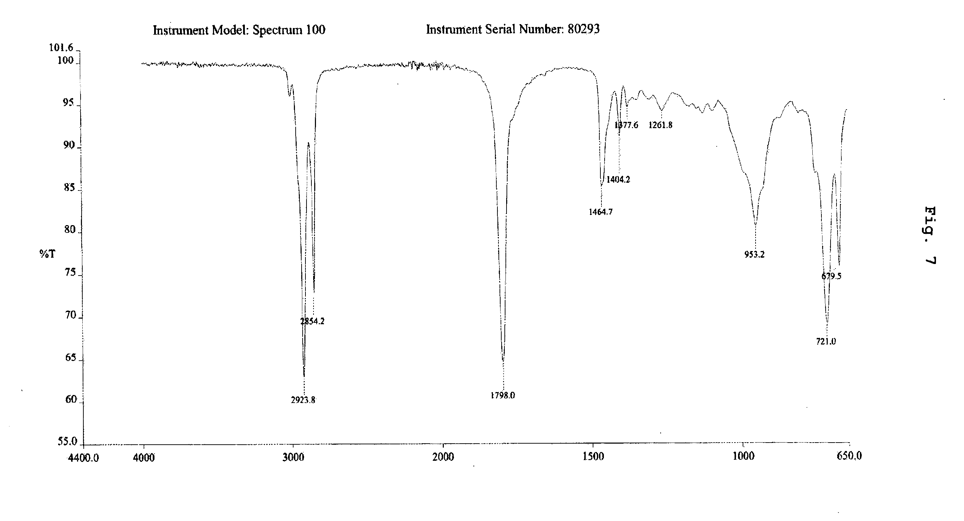

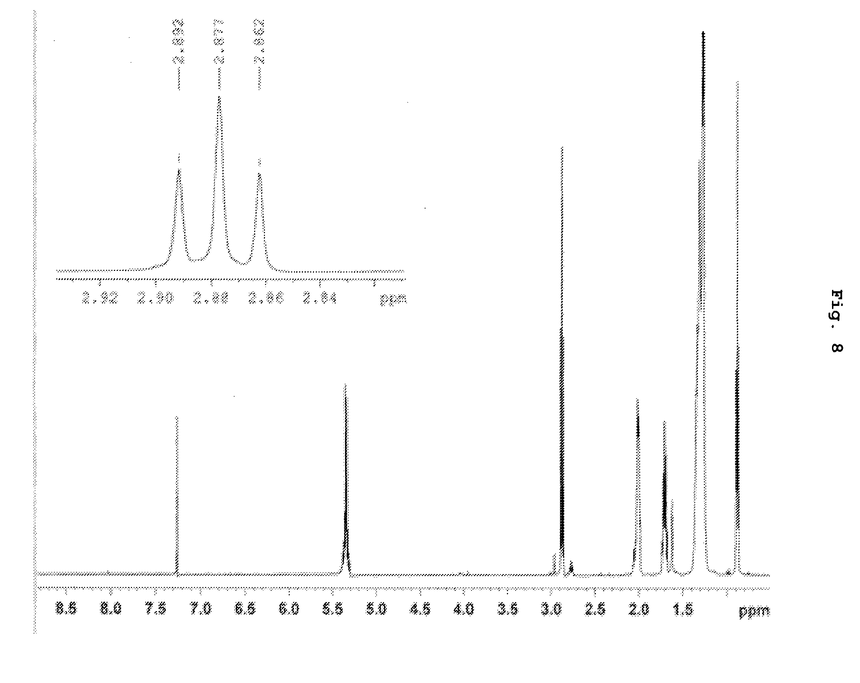

Dans le but d'analyser la composition du mélange de chlorures d'acides gras obtenus, quelques microlitres de distillat sont exposés à du méthanol. Le totum est alors injecté sur un chromatographe en phase gazeuse équipé d'une colonne de type « FAME » (Fatty Acid Methyl Ester) et d'un détecteur de masse en ligne (DSQ-II). Dans le chromatogramme présenté en Figure 6, le pic à 17,8 min correspond au stéarate (M+˙ = 298), celui à 18,07 min à l'oléate (M+˙ = 296), celui à 18,08 min à un linoléate (M+˙ = 294) et celui à 19,38 min au linolénate (M+˙ = 292). Leurs intensités relatives sont une bonne indication de leurs proportions respectives.
Les spectres IR-FT (Fig. 7) et de RMN du proton (Fig. 8) sont en parfait accord avec la formation exclusive de ces chlorures :
Une bande à 1798 cm⁻¹, caractéristique des chlorures d'acyles.
Les protons en alpha du carbonyle (t, *J*= 7,5 Hz), présentent un déplacement chimique à 2,9 ppm, caractéristique de la transformation des carboxyles en chlorures d'acides.

### Exemple 5 : Estérification des oligomères de resvératrol O-alkylés

8,4 g (35 mmoles) d'oligomères de resvératrol O-alkylés selon l'exemple 3, sont mis en suspension dans 106 ml d'hexane additionné de 9,3 ml de triéthylamine (70 mmoles),sont agités sous atmosphère d'argon. On ajoute goutte à goutte, 10,65 g des chlorures préparés à l'exemple 4, dilués dans 45 mL d'hexane (35,1 mmoles, 1 éq.).

La réaction est laissée encore 6 heures sous agitation à température ambiante, avant d'être placée en ampoule à décanter et lavée par 100 mL d'acide chlorhydrique au dixième, puis 90 mL d'une solution de NaHCO₃ à 10% (p/v) dans l'eau, et enfin, par de l'eau distillée jusqu'à neutralité (deux fois 90 mL). La phase organique est séchée sur sulfate de sodium, filtrée, puis évaporée à siccité, sous pression réduite. Elle abandonne un résidu de 19,21 g d'actifs stilbénoïdiques de sarments de vigne alkylés et stabilisés (= 24,64 mmoles ; rendement brut = 70,6 %, pm moyen = 774).

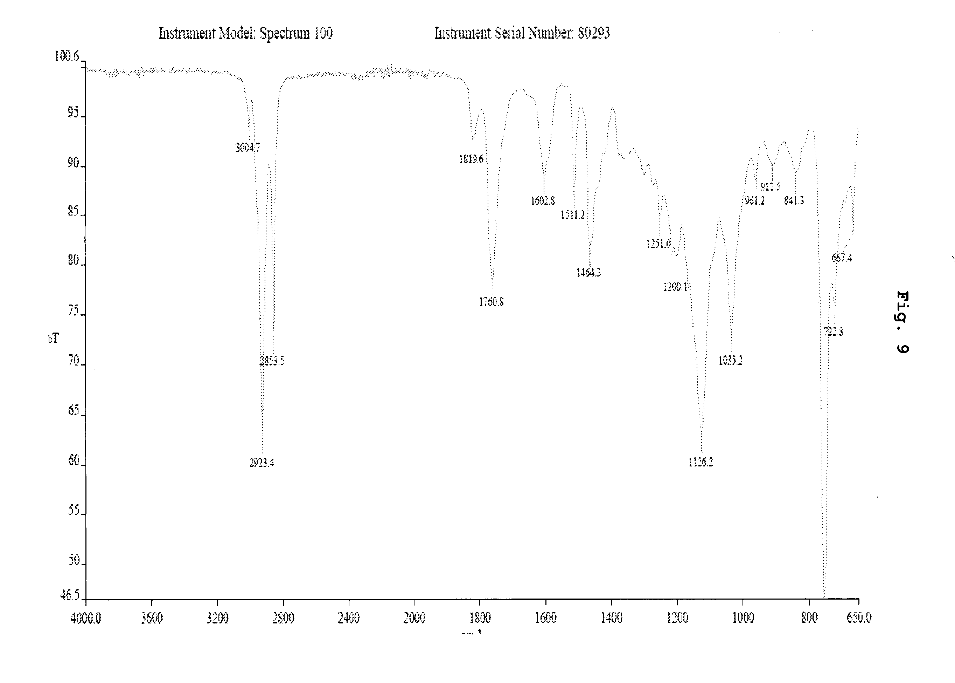

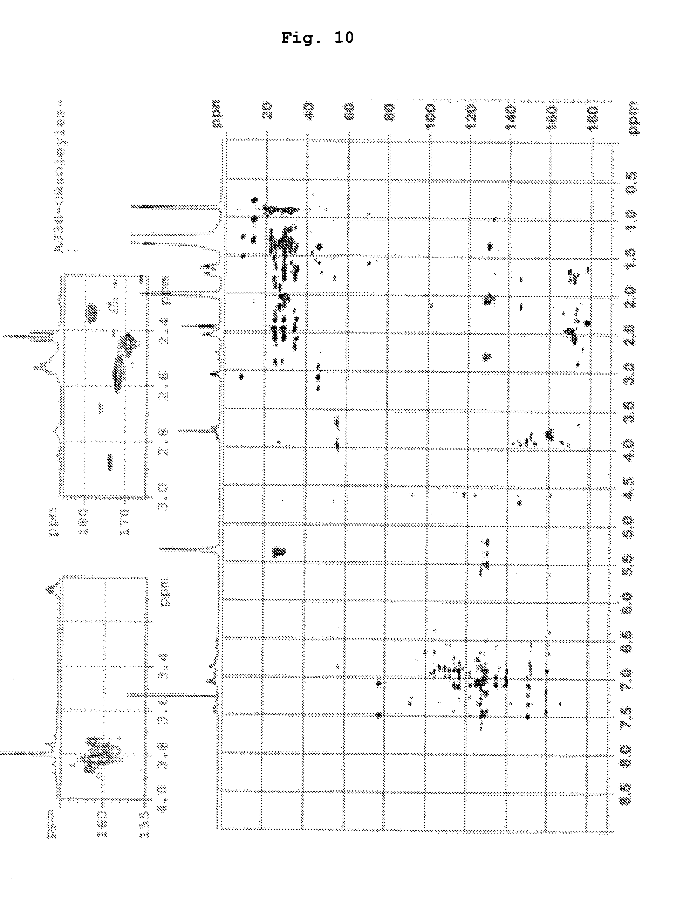

Dans le but d'obtenir des moyens d'identification de ces actifs, le totum est alors soumis à des mesures spectrales :
- Le spectre infrarouge par transformée de Fourier acquis en mode ATR (Fig. 9), montre l'apparition d'une bande intense à 1764 cm⁻¹, caractéristique des carboxyles d'esters phénoliques, concomitante de la disparition de la bande large centrée sur 3350 cm⁻¹, qui correspondait aux hydroxyles phénoliques libres.
- Le spectre de RMN bidimensionnelle hétéronucléaire ¹H-¹³C à longue distance à 500 MHz (Fig. 10), obtenu en mode inverse (HMBC), fait apparaître nettement, les corrélations qui sont en parfait accord avec les structures diversifiées de polyphénols stilbénoïdiques alkylés (éthers méthyliques d'oxygènes aromatiques) et estérifiés (esters d'acides gras majoritairement insaturés, en mélange statistique tel que résultant de l'huile d'olive utilisée pour préparer les agents d'acylation).

Dans le cas préféré, où chaque molécule de l'extrait initial n'a subi qu'une méthylation par unité stilbénoïdique (« resvératrol »), et où les fonctions phénoliques résiduelles sont toutes acylées par le mélange d'AG d'huile d'olive, on obtient un mélange des différents régio- et stéréo-isomères possibles de monomères et dimères figurés ci-dessous:

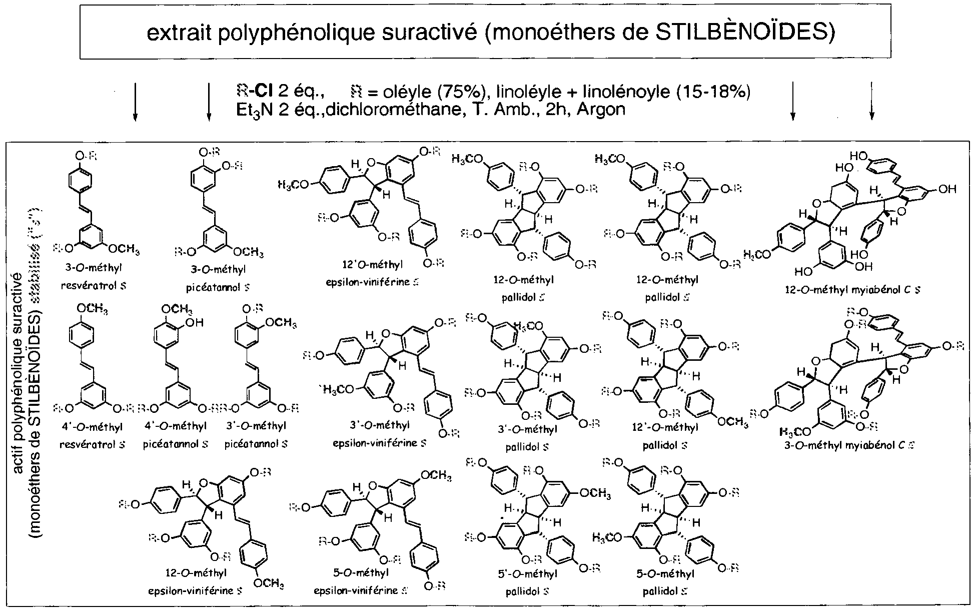

### Exemple 5 : Formulations cosmétiques

### - FORMULE A

| **PHASES** | **MATIERES PREMIERES** | **%** |
|---|---|---|
| 101 | Eau | 80,8000 |
| 102 | EDTA Tétrasodique | 0,0500 |
| 103 | Glycérine | 5,0000 |
| 104 | Carbomère | 0,3500 |
| | | |
| 201 | Cétéaryl Glycosides de blé | 0,7500 |
| 202 | Cétéaryl Glycosides d'orge | 1,7500 |
| 203 | Alcool Cétéarylique | 2,5000 |
| **204** | **Composition de l'invention** | **0,05 à 1** |
| 205 | Beurre de Butyrospermum Parkii | 2,5000 |
| 206 | Acétate de tocophéryle | 0,5000 |
| 207 | Huile de pépins de raisin (*Vitis Vinifera*) | 3,0000 |
| 208 | Alcool cétylique | 1,0000 |
| 209 | Cétyl phosphate de potassium | 1,0000 |
| | | |
| 301 | Conservateurs | 0,6000 |
| | | |
| 401 | Fragrance | |
| | | 0,2000 |
| 501 | Hydroxyde de sodium Qps pH 6,00 | |

### - FORMULE B

| **PHASES** | **MATIERES PREMIERES** | **%** |
|---|---|---|
| 101 | Eau | 79,40000 |
| 102 | EDTA Tétrasodique | 0,05000 |
| 103 | Acide Citrique Qsp pH final 5,5 | 0,15000 |
| | | |
| 201 | Gomme Xanthique | 0,30000 |
| 202 | Butylène Glycol | 5,00000 |
| | | |
| 301 | Cétéareth-20 | 1,50000 |
| 302 | Stéarate de glycéryle | 2,00000 |
| 303 | **Composition de l'invention** | **0,05 à 1** |
| 304 | Beurre de Butyrospermum Parkii | 1,00000 |
| 305 | Laurate d'hexyle | 4,00000 |
| 306 | Diméthicone | 3,00000 |
| 307 | Squalane | 2,00000 |
| 308 | Acétate de tocophéryle | 0,50000 |
| | | |
| 401 | Conservateurs | 0,60000 |
| | | |
| 501 | Fragrance | |
| | | 0,50000 |

## Revendications

1. Compositions de polyphénols, **caractérisées en ce qu'**il s'agit de monomères, d'oligomères ou de polymères unités polyphénoliques répondant à la formule (I) : 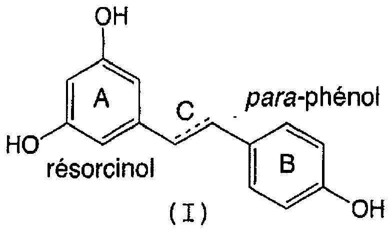 ces unités étant **caractérisées par** la présence simultanée d'un noyau de type résorcinol (noyau A) et d'un noyau de type para-phénol (noyau B), reliés entre eux par un lien carboné C, lesdites unités étant suractivées, en ce qui concerne leur pouvoir nucléophile, par alkylation d'au moins une fonction phénolique de chaque unité présente dans des monomères, des oligomères ou des polymères, et par estérification par des mélanges d'acides gras comprenant de manière majoritaire des acides gras insaturés (AGI) des fonctions phénoliques non modifiées par l'alkylation.

2. Compositions selon la revendication 1, **caractérisées en ce que** dans lesdites unités les noyaux A et B sont confondus et le segment C est inexistant, comme dans le phloroglucinol de formule (II)

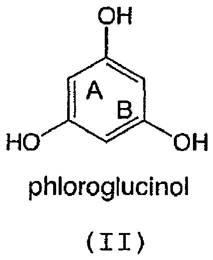

3. Compositions selon la revendication 1, **caractérisées en ce que**, dans lesdites unités, les noyaux A et B sont distincts et le segment C est constitué de 2 carbones qui sont soit hybridés sp2 et formant un vinyle comme dans le resvératrol de formule (III) 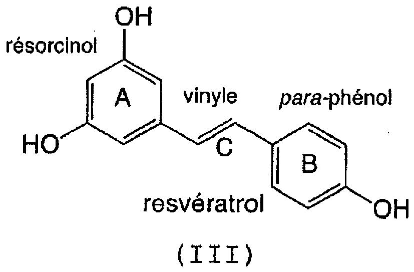 soit hybridés sp3 et servant de point d'attachement entre les monomères pour former les oligomères et polymères.

4. Compositions selon l'une quelconque des revendications 1 à 3, **caractérisées en ce que** le nombre de groupes -*O*-alkyles n'égale pas le nombre d'hydroxyles présents en moyenne par unité polyphénolique constitutive de monomères, d'oligomères ou de polymères.

5. Compositions selon l'une quelconque des revendications 1 à 4, **caractérisées en ce que** le ou les groupes alkyles sont des groupes méthyles, isopropyles ou *tert*-butyles.

6. Compositions selon l'une quelconque des revendications 1 à 5, **caractérisées en ce que** lesdits esters sont des esters d'acides gras d'huiles végétales, comprenant les radicaux R d'acyles correspondant aux acides gras saturés, comme l'acide stéarique, aux acides gras monoinsaturés, comme l'acide oléique, et aux acides gras polyinsaturés essentiels, comme les acides linoléique et linolénique.

7. Compositions selon la revendication 6, **caractérisées en ce que** les huiles végétales sont choisies parmi l'huile d'olive ou de pépins de raisin.

8. Compositions selon l'une quelconque des revendications 1, 2 ou 4 à 7, **caractérisées en ce que** lesdits polyphénols répondent à la formule (IV) 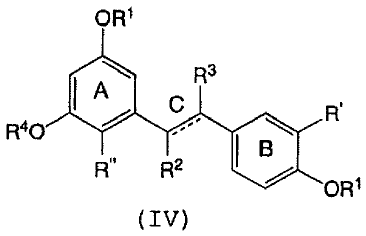 dans laquelle
- R¹ est un radical alkyle, ou un radical acyle d'un acide gras d'une huile végétale, représenté par R tel que défini dans la revendication 6,
- R² est un hydrogène ou le point de jonction en R" ou à R² d'une autre unité,
- -R³ est un hydrogène ou le point de jonction en R" ou en R⁴ d'une autre unité,
- R⁴ est un radical alkyle, ou un radical acyle d'un acide gras d'une huile végétale, représente par R tel que défini dans la revendication 6 ou le point de jonction en R³ d'une autre unité.
- R" représente H ou le point de jonction en R² ou en R³ d'une autre unité,
- R' est un hydrogène ou un radical O-acyle d'un acide gras d'une huile végétale, représenté par R tel que défini ci-dessus
et les diastéréoisomères et les régioisomères de ces motifs.

9. Compositions selon la revendication 8, **caractérisées en ce que** lesdits polyphénols correspondent aux dimères et trimères de formule, respectivement, V et VI

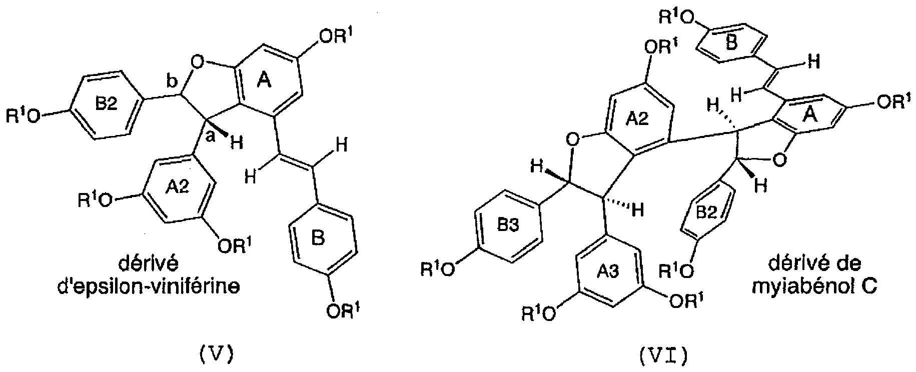

10. Compositions selon l'une quelconque des revendications 1 à 9, **caractérisées en ce que** lesdits polyphénols correspondent à des dérivés alkylés et estérifiés d'extraits végétaux.

11. Compositions selon la revendication 10, **caractérisées en ce que** lesdits extraits végétaux sont des extraits de vigne, en particulier, ces extraits comprenant des dérivés polyphénoliques constituant des vinylogues du phloroglucinol, notamment le resvératrol, le picéatannol, l'epsilon-viniférine, le pallidol, le miyabénol C, correspondant respectivement, aux formules III, VII, VIII, IX, et X ci-dessous : 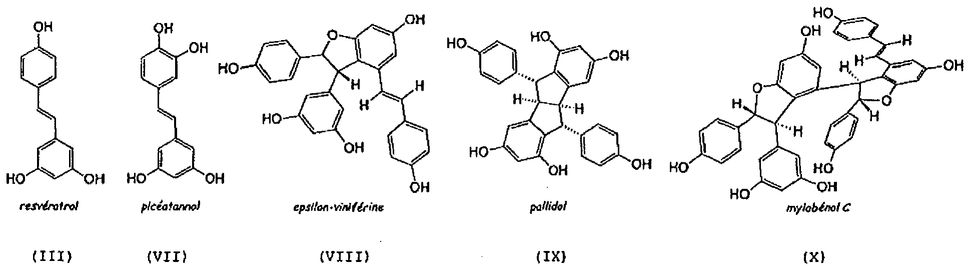 obtenus à partir de sarments et/ou de rafles de vigne, de Polygonum, de fruits, par exemple de mûriers.

12. Procédé de préparation de compositions selon l'une quelconque des revendications 1 à 11, **caractérisé en ce qu'**on fait réagir les polyphénols des extraits végétaux définis ci-dessus
- dans une première étape, avec un agent d'alkylation dans des conditions permettant de substituer l'hydrogène d'au moins 1 groupe OH phénolique par unité polyphénolique constitutive de monomères, d'oligomères ou de polymères, de préférence de 1 à 2, par un groupe alkyle, et
- dans une deuxième étape, avec un agent d'acylation, notamment, un anhydride ou un chlorure d'acide, dans des conditions permettant de substituer l'hydrogène des fonctions phénoliques laissées intactes par l'alkylation, par un mélange de radicaux acyles, R étant tel que défini dans la revendication 6.

13. Compositions cosmétiques, **caractérisées en ce qu'**elles renferment une quantité efficace pour lutter contre le vieillissement de la peau, d'une ou plusieurs compositions desdits polyphénols selon l'une quelconque des revendications 1 à 11, en association avec des véhicules inertes appropriés pour un usage externe.

14. Application des compositions selon l'une quelconque des revendications 1 à 11, en diététique.

15. Compositions selon l'une quelconque des revendications 1 à 11, pour une utilisation comme médicaments.

## Patentansprüche

1. Zusammensetzungen von Polyphenolen, **dadurch gekennzeichnet, dass** es sich um Monomere, um Oligomere oder um Polymere von Polyphenoleinheiten handelt, die der Formel (I) entsprechen: 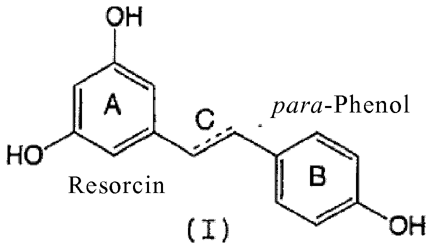 wobei diese Einheiten **gekennzeichnet sind durch** die gleichzeitige Anwesenheit eines Kerns vom Typ Resorcin (Kern A) und eines Kerns vom Typ para-Phenol (Kern B), die **durch** eine Kohlenstoffbindung C untereinander verbunden sind, wobei die Einheiten, was ihre nukleophile Fähigkeit anbelangt, **durch** Alkylierung wenigstens einer Phenolfunktion einer jeden Einheit, welche in Monomeren, Oligomeren oder Polymeren vorliegt, sowie **durch** Veresterung mit Fettsäuremischungen, die überwiegend ungesättigte Fettsäuren (UFS) der nicht **durch** die Alkylierung modifizierten Phenolfunktionen umfassen, überaktiviert werden.

2. Zusammensetzungen nach Anspruch 1, **dadurch gekennzeichnet, dass** bei den Einheiten die Kerne A und B verschmolzen sind und das Segment C nicht vorhanden ist, wie bei dem Phloroglucin der Formel (II)

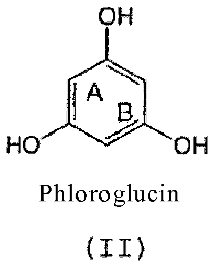

3. Zusammensetzungen nach Anspruch 1, **dadurch gekennzeichnet, dass** bei den Einheiten die Kerne A und B getrennt sind und das Segment C aus 2 Kohlenstoffen besteht, die entweder sp2-hybridisiert sind und ein Vinyl bilden, wie bei dem Resveratrol der Formel (III) 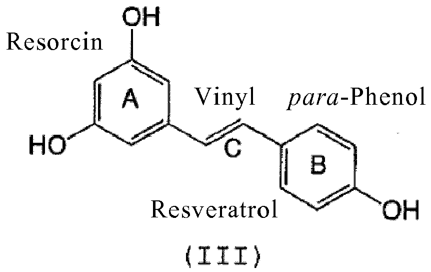 oder sp3-hybridisiert sind und als Bindungsstelle zwischen den Monomeren dienen, um die Oligomere und Polymere zu bilden.

4. Zusammensetzungen nach einem der Ansprüche 1 bis 3, **dadurch gekennzeichnet, dass** die Anzahl an -O-Alkyl-Gruppen nicht gleich der Anzahl an Hydroxylen ist, die im Durchschnitt pro konstitutiver Polyphenoleinheit von Monomeren, Oligomeren oder Polymeren vorhanden sind.

5. Zusammensetzungen nach einem der Ansprüche 1 bis 4, **dadurch gekennzeichnet, dass** die Alkylgruppe oder -gruppen Methyl-, Isopropyl- oder *tert-*Butylgruppen sind.

6. Zusammensetzungen nach einem der Ansprüche 1 bis 5, **dadurch gekennzeichnet, dass** die Ester Fettsäureester aus pflanzlichen Ölen sind, umfassend die Acylreste R, die den gesättigten Fettsäuren, wie Stearinsäure, den einfach ungesättigten Fettsäuren, wie Ölsäure, und den mehrfach ungesättigten essentiellen Fettsäuren, wie Linolsäure und Linolensäure, entsprechen.

7. Zusammensetzungen nach Anspruch 6, **dadurch gekennzeichnet, dass** die pflanzlichen Öle aus Olivenöl oder aus Traubenkernöl ausgewählt sind.

8. Zusammensetzungen nach einem der Ansprüche 1, 2 oder 4 bis 7, **dadurch gekennzeichnet, dass** die Polyphenole der Formel (IV) entsprechen 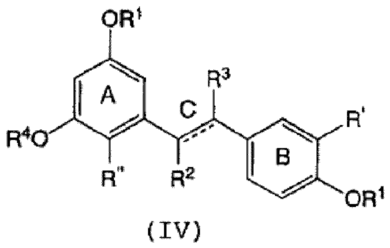 worin
- R¹ ein Alkylrest oder ein Acylrest einer Fettsäure eines pflanzlichen Öls ist, dargestellt durch R, wie in Anspruch 6 definiert,
- R² ein Wasserstoff oder die Verbindungsstelle an R" oder mit R² einer weiteren Einheit ist,
- -R³ ein Wasserstoff oder die Verbindungsstelle an R" oder an R⁴ einer weiteren Einheit ist,
- R⁴ ein Alkylrest oder ein Acylrest einer Fettsäure eines pflanzlichen Öls, dargestellt durch R, wie in Anspruch 6 definiert, oder die Verbindungsstelle an R³ einer weiteren Einheit ist,
- R" H oder die Verbindungsstelle an R² oder an R³ einer weiteren Einheit darstellt,
- R' ein Wasserstoff oder ein O-Acyl-Rest einer Fettsäure eines pflanzlichen Öls ist, dargestellt durch R, wie oben definiert
und die Diastereoisomere und die Regioisomere dieser Muster.

9. Zusammensetzungen nach Anspruch 8, **dadurch gekennzeichnet, dass** die Polyphenole den Dimeren und Trimeren der Formel V bzw. VI entsprechen

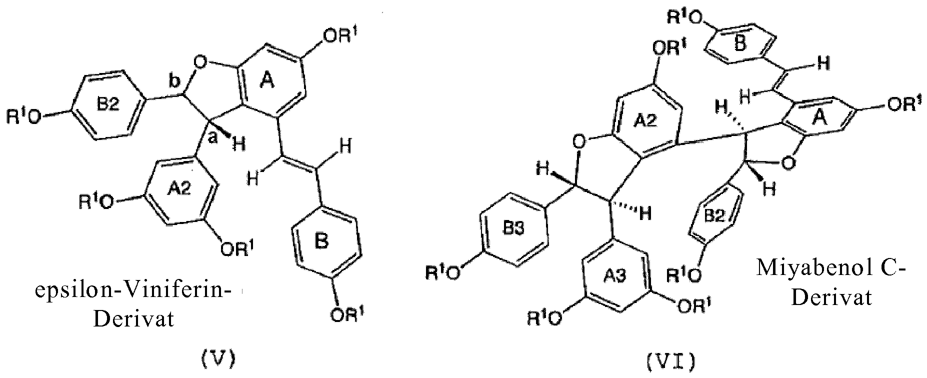

10. Zusammensetzungen nach einem der Ansprüche 1 bis 9, **dadurch gekennzeichnet, dass** die Polyphenole alkylierten und veresterten Derivaten von Pflanzenextrakten entsprechen.

11. Zusammensetzungen nach Anspruch 10, **dadurch gekennzeichnet, dass** die Pflanzenextrakte insbesondere Rebenextrakte sind, wobei diese Extrakte Polyphenol-Derivate umfassen, die Vinyloge des Phloroglucin bilden, insbesondere Resveratrol, Piceatannol, epsilon-Viniferin, Pallidol, Miyabenol C, welche den nachstehenden Formeln III, VII, VIII, IX bzw. X entsprechen: 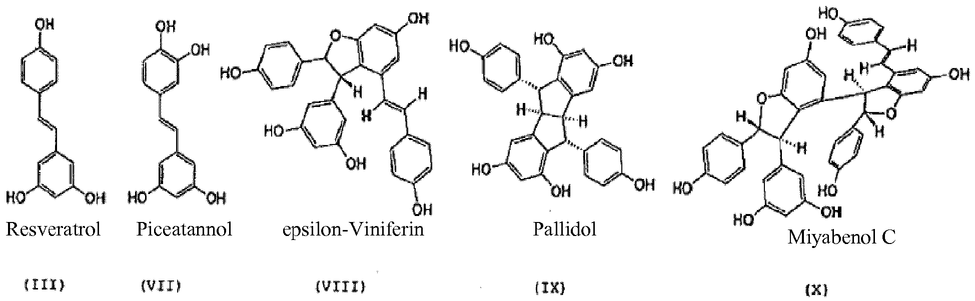 die aus Weinranken und/oder -rappen, Polygonum, Früchten, beispielsweise von Maulbeerbäumen erhalten werden.

12. Verfahren zur Zubereitung von Zusammensetzungen nach einem der Ansprüche 1 bis 11, **dadurch gekennzeichnet, dass** man die Polyphenole der Pflanzenextrakte, welche oben definiert sind,
- in einem ersten Schritt mit einem Alkylierungsmittel unter Bedingungen reagieren lässt, die ermöglichen, den Wasserstoff wenigstens 1 OH-Phenolgruppe pro konstitutiver Polyphenoleinheit von Monomeren, Oligomeren oder Polymeren, vorzugsweise 1 bis 2, durch eine Alkylgruppe zu ersetzen, und
- in einem zweiten Schritt mit einem Acylierungsmittel, insbesondere einem Säureanhydrid oder -chlorid unter Bedingungen reagieren lässt, die ermöglichen, den Wasserstoff der durch die Alkylierung intakt gelassenen Phenolfunktionen durch eine Mischung von Acylresten zu ersetzen, wobei R so ist wie in Anspruch 6 definiert.

13. Kosmetische Zusammensetzungen, **dadurch gekennzeichnet, dass** sie eine wirkungsvolle Menge zum Bekämpfen der Hautalterung, von einer oder mehreren Zusammensetzungen der Polyphenole nach einem der Ansprüche 1 bis 11, in Verbindung mit für eine äußere Anwendung geeigneten inerten Trägerstoffen enthalten.

14. Anwendung der Zusammensetzungen nach einem der Ansprüche 1 bis 11, in der Diätetik.

15. Zusammensetzungen nach einem der Ansprüche 1 bis 11 für eine Verwendung als Arzneimittel.

## Claims

1. Compositions of polyphenols, **characterized in that** they are monomers, oligomers or polymers of polyphenolic units conforming to the formula (I): 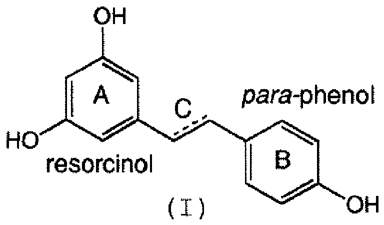 these units being **characterized by** the simultaneous presence of a resorcinol type nucleus (nucleus A) and of a para-phenol type nucleus (nucleus B), which are joined to one another by a carbon linkage C, said derivatives being overactivated, with regard to their nucleophilic power, by alkylation of at least one phenolic function of each unit present in monomers, oligomers or polymers, and by esterification with mixtures of predominantly unsaturated fatty acids (UFA) of the phenolic functions non modified by the alkylation.

2. The compositions according to claim 1, **characterized in that** in said units the nuclei A and B are merged and the segment C is absent, as in phloroglucinol of formula (II):

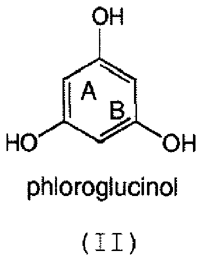

3. The compositions according to Claim 1, **characterized in that**, in said units, the nuclei A and B are distinct and the segment C is composed of 2 carbons which are either sp2 hybridized and form a vinyl, as in resveratrol of formula (III): 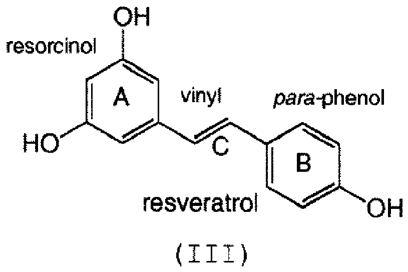 or segment C is composed of sp3 hybridized carbons and serves as a point of attachment between the monomers for forming the oligomers and polymers.

4. The compositions according to anyone of claims 1 to 3, **characterized in that** the number of -*O*-alkyl groups per unit is not equal to the number of hydroxyls present on average per constituent polyphenolic unit of monomers, oligomers or polymers.

5. The compositions according to anyone of claims 1 to 4, **characterized in that** the alkyl group(s) is (are) methyl, isopropyl or *tert*-butyl groups.

6. The compositions according to anyone of claims 1 to 5, **characterized in that** said esters are fatty acid esters of vegetable oils, comprising the acyl radicals R corresponding to saturated fatty acids, such as stearic acid, to monounsaturated fatty acids, such as oleic acid, and to essential polyunsaturated fatty acids, such as linoleic and linolenic acids.

7. The compositions according to claim 6, **characterized in that** the vegetable oils are selected from olive oil or grapeseed oil.

8. The compositions according to anyone of claims 1, 2, and 4 to 7, **characterized in that** said polyphenols conform to the formula (IV): 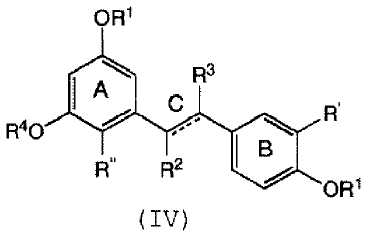 in which
- R¹ is an alkyl radical, or an acyl radical of a fatty acid of a vegetable oil, represented by R as defined in claim 6,
- R² is a hydrogen or the junction point at R" or to R² of another unit,
- R³ is a hydrogen or the junction point at R" or at R⁴ of another unit,
- R⁴ is an alkyl radical, or an acyl radical of a fatty acid of a vegetable oil, represented by R as defined in claim 6, or the junction point at R³ of another unit,
- R" represents H or the junction point at R² or at R³ of another unit,
- R' is a hydrogen or an O-acyl radical of a fatty acid of a vegetable oil, represented by R as defined above
and the diastereoisomers and regioisomers of these moieties.

9. The compositions according to claim 8, **characterized in that** said derivatives correspond to the dimers and trimers of formula V and VI respectively:

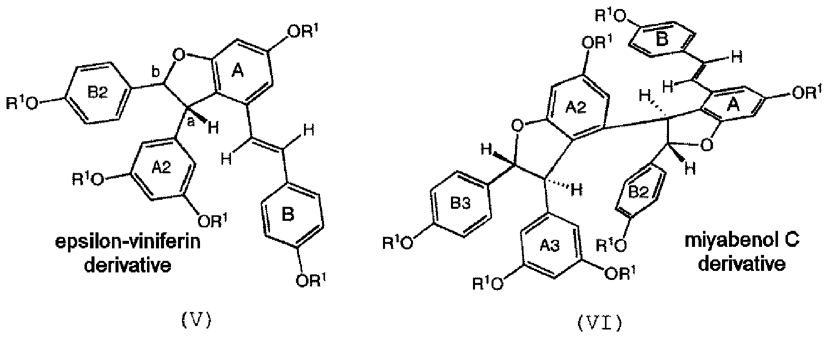

10. The compositions according to anyone of claims 1 to 9, **characterized in that** said polyphenols derivatives correspond to alkylated and esterified derivatives of plant extracts.

11. The compositions according to claim 10, **characterized in that** said plant extracts are vine extracts, particularly said extracts comprise polyphenol derivatives which constitute vinylogues of phloroglucinol, especially resveratrol, piceatannol, epsilon-viniferin, pallidol, miyabenol C, corresponding respectively to the formulae III, VII, VIII, IX, and X below: 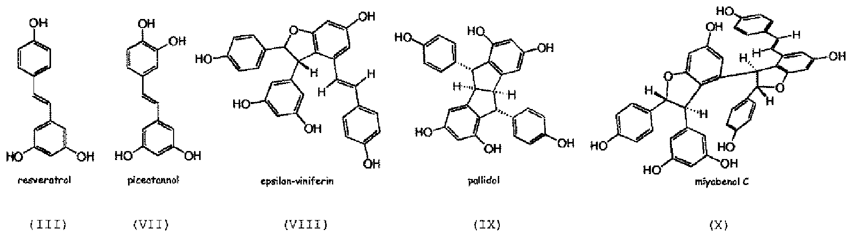 obtained from vine shoots and/or stems, Polygonum, fruit, from mulberry plants, for example.

12. A process for preparing compositions according to anyone of claims 1 to 11, **characterized in that** the plant extract polyphenols defined above are reacted
- in a first step, with an alkylating agent under conditions allowing substitution of an alkyl group for the hydrogen of at least 1 phenolic OH group per polyphenolic constituent unit of monomers, oligomers or polymers, preferably of 1 to 2, and
- in a second step, with an acylating agent, especially an acid anhydride or acid chloride, under conditions allowing substitution for hydrogen of the phenolic functions still intact after the alkylation, by a mixture of acyl radicals, R being as defined in claim 6.

13. Cosmetic compositions, **characterized in that** they comprise an amount effective for combating skin aging of one or more compositions of polyphenol derivatives according to any of claims 1 to 11, in combination with inert vehicles appropriate for external use.

14. The application of the compositions according to anyone of claims 1 to 11 in dietetics.

15. The compositions according to anyone of claims 1 to 11 for use as medicaments.
